# EUROPEAN PATENT APPLICATION

(11) **EP 2 436 776 A1**
(43) Date of publication of application: **04.04.2012**
(21) Application number: 10185418.0
(22) Date of filing: 01.10.2010
(51) Int. Cl.: C12Q 1/37, G01N 33/573, G01N 33/569

(54) **Method for monitoring Cathepsin S inhibition**

(71) Applicant: F. Hoffmann-La Roche AG, 4070 Basel (CH)
(72) Inventor: The designation of the inventor has not yet been filed
(74) Representative: Küng, Peter

(57) **Abstract**

The present invention relates to a method for monitoring Cathepsin S inhibitor activity in tissue samples of animals comprising:
a) providing a tissue sample of an animal to whom a Cathepsin S inhibitor has been administered or providing a tissue sample of an animal that was contacted *in vitro* with a Cathepsin S inhibitor, wherein the tissue samples comprise white blood cells,
b) measuring CLIP peptide level and/or MHC II polypeptide level in the white blood cells of the tissue sample of step a) and
c) correlating CLIP peptide level and/or MHC II polypeptide level in the white blood cells to Cathepsin S inhibitor dose, wherein a Cathepsin S inhibitor leads to decreased level of CLIP peptide and/or a decreased level of MHC II polypeptide in the white blood cells.

## Description

The present invention provides a method for monitoring Cathepsin S inhibition and a method for screening of compounds for Cathepsin S inhibitory activity.

Cathepsin S is well known for its critical function in the proteolytic digestion of the invariant chain chaperone molecules, thus controlling antigen presentation to CD4⁺ T-cells by major histocompatibility complex (MHC) class II molecules or to NK1.1+ T-cells via CD1 molecules. Cathepsin S also appears to participate in direct processing of exogenous antigens for presentation by MHC class II to CD4⁺ T-cells, or in cross-presentation by MHC class I molecules to CD8⁺ T-cells. In addition, in its secreted form cathepsin S is implicated in degradation of the extracellular matrix, which may contribute to the pathology of a number of diseases, including arthritis, atherosclerosis and chronic obstructive pulmonary disease. Therefore, inhibition of cathepsin S is a promising target for the development of novel therapeutics for a variety of indications.

MHC class II molecules bind and display antigenic peptides as class II-peptide complexes on the cell surface of antigen presenting cells (APCs) for recognition by CD4⁺ helper cells. The molecular mechanisms leading to formation of class II-peptide complexes and presentation of antigen on the cell surface begin with synthesis of class II αβ heterodimers in the endoplasmatic reticulum. During biosynthesis these αβ heterodimers associate with a type II membrane protein, the invariant chain li, to form a nonameric complex of αβli. The αβli complex traverses the Golgi apparatus and is delivered to intracellular compartments. In these acidic compartments, the li is degraded and liberated from the αβli complexes, allowing class II molecules to bind antigenic peptides. The cysteine protease Cathepsin S plays a key role in processing li in APCs. Cathepsin S degrades the invariant chain li from class II-li complexes generating class II-CLIP. Inhibition of Cathepsin S leads to a decrease of CLIP peptide and to accumulation of invariant chain li degradation peptide p10.

EP 1 315 966 discloses a method for monitoring the effect of in vivo administration of cathepsin S inhibitors. The method includes the purification of white blood cells from a blood sample of a subject treated with a cathepsin S inhibitor, preparation of whole cell lysates of the purified white blood cells and analyzing the whole cell lysates for presence of p10 fragment of invariant chain li. This method requires blood samples of about 10 - 30 ml and is labour intensive (isolation of white blood cells and preparation of whole cell lysates).

Therefore, there is a need for a cathepsin S inhibitor assay which overcomes at least some of the drawbacks of the prior art methods.

In a first object, the present invention provides a method for monitoring Cathepsin S inhibitor activity in tissue samples of animals comprising:
a) providing a tissue sample of an animal to whom a Cathepsin S inhibitor has been administered or providing a tissue sample of an animal that was contacted *in vitro* with a Cathepsin S inhibitor, wherein the tissue sample comprises white blood cells,
b) measuring CLIP peptide level and/or MHC II polypeptide level in the white blood cells of the tissue sample of step a) and
c) correlating CLIP peptide level and/or MHC II polypeptide level in the white blood cells to Cathepsin S inhibitor dose, wherein a Cathepsin S inhibitor leads to decreased level of CLIP peptide and/or a decreased level of MHC II polypeptide in the white blood cells.

In a second object the present invention provides a method for the identification of a Cathepsin S inhibitor comprising:
a) treating a non-human animal with a test compound,
b) providing a tissue sample of the animal of step a) comprising white blood cells,
c) measuring CLIP peptide level and/or MHC II polypeptide level in the white blood cells of the tissue sample of step b),
   wherein a decreased level of CLIP peptide and/or a decreased level of MHC II polypeptide in the white blood cells in the sample of step a) compared to a level of CLIP peptide and/or a level of MHC II polypeptide in white blood cells in a tissue sample of an untreated animal is indicative for a Cathepsin S inhibitor.

In a preferred embodiment of the methods of the present invention the tissue sample is blood.

In a further preferred embodiment of the methods of the present invention the level of CLIP peptide and/or MHC II polypeptide level on the cell surface of white blood cells is measured.

In a further preferred embodiment of the methods of the present invention the white blood cells are Antigen Presenting Cells (APCs), preferably the APCs are selected from B cells, monocytes, macrophages and dendritic cells, more preferably selected from B cells and monocytes.

In another preferred embodiment of the methods of the present invention the level of CLIP peptide and/or MHC II polypeptide on the cell surface of white blood cells, preferably APCs, is measured by Flow Cytometry.

The term Antigen Presenting cells (APCs) as used herein refers to cells that display antigenic peptides as (MHC) class II - antigenic peptide complex on its surface. APCs comprise dendritic cells, monocytes, macrophages and B cells.

The term Cathepsin S refers to a cysteine protease in the papain super-family. The amino acid sequence of human Cathepsin S is given in Seq. Id. No. 1.

The term "invariant chain li" refers to invariant polypeptide of major histocompatibility complex or CD74. The amino acid sequence of human invariant chain li is given in Seq. Id. No. 2.

The term CLIP refers to class II-associated li peptide. The amino acid sequence of human CLIP is given in Seq. Id. No. 3.

The term "MHC II polypeptide" as used herein refers to the alpha chain and the beta chain constituting the MHC II complex.

The term animal as used herein comprises human beings and non-human animals such as e.g. monkey, dog, pig, rabbit, guinea pig and rodents.

The term "compound" is used herein in the context of a "test compound" or a "drug candidate compound" described in connection with the assays of the present invention. As such, these compounds comprise organic or inorganic compounds, derived synthetically or from natural sources. The compounds include inorganic or organic compounds such as polynucleotides, lipids or hormone analogs that are characterized by relatively low molecular weights. Other biopolymeric organic test compounds include peptides comprising from about 2 to about 40 amino acids and larger polypeptides comprising from about 40 to about 500 amino acids, such as antibodies or antibody conjugates.

The inventors of the present invention have surprisingly found that CLIP peptide can be detected on the cell surface of white blood cells and not only intracellularly and that Cathepsin S inhibitors decrease the expression of MHC II polypeptides on the cell surface of white blood cells. The methods of the present invention have the advantage compared to the methods described in the prior art that tissue samples, preferably whole blood, can be assayed directly without involving the isolation of white blood cells. In the methods of the present invention there is no need for the isolation of e.g. white blood cells from whole blood samples, in order to be able to be processed in a method of the present invention. This is of great importance if the assay is used in clinical settings where a large number of samples have to be processed. The methods of the present invention require smaller tissue samples, preferably blood samples, than the prior art methods.

Methods for detection and/or measurement of polypeptides in biological samples are well known in the art and include, but are not limited to, Western-blotting, Flow cytometry, ELISAs or RIAs, or various proteomics techniques. Monoclonal or polyclonal antibodies recognizing the CLIP peptide or MHC II polypeptide or fragments thereof, can either be generated for the purpose of detecting the polypeptides or peptide fragments, e.g. by immunizing rabbits with purified proteins, or known antibodies recognizing the polypeptides or peptide fragments can be used. For example, an antibody capable of binding to the denatured proteins, such as a polyclonal antibody, can be used to detect CLIP peptide or MHC II polypeptide in a Western Blot. An example for a method to measure a polypeptide is an ELISA. This type of protein quantitation is based on an antibody capable of capturing a specific antigen, and a second antibody capable of detecting the captured antigen. Methods for preparation and use of antibodies, and the assays mentioned hereinbefore are described in Harlow, E. and Lane, D. Antibodies: A Laboratory Manual, (1988), Cold Spring Harbor Laboratory Press.

A preferred method for the detection of CLIP peptide and/or MHC II polypeptide is flow cytometry. Flow cytometry methods are described in Handbook of Flow Cytometry Method, J. Paul Robinson (Editor); Flow Cytometry - A Basic Introduction, Michael G Ormerod (2008) and Current Protocols in Cytometry (2010), Wiley.

### Short description of the figures

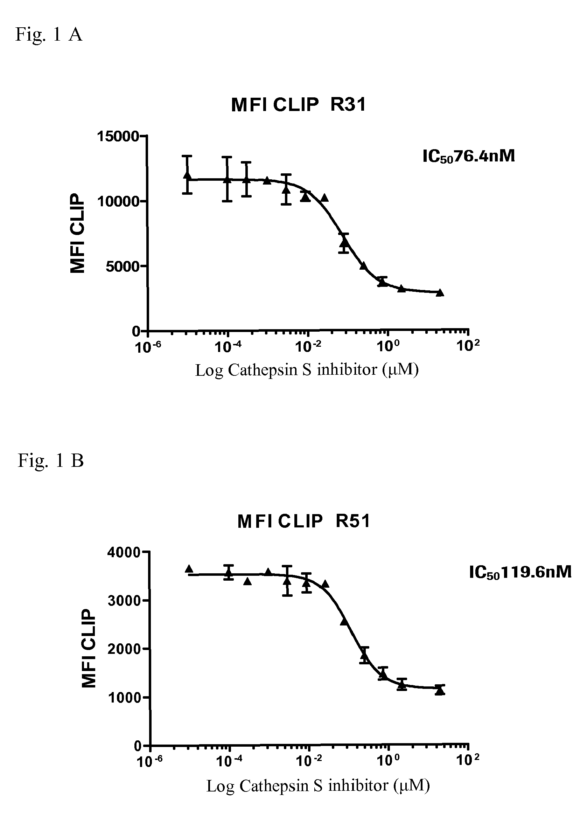

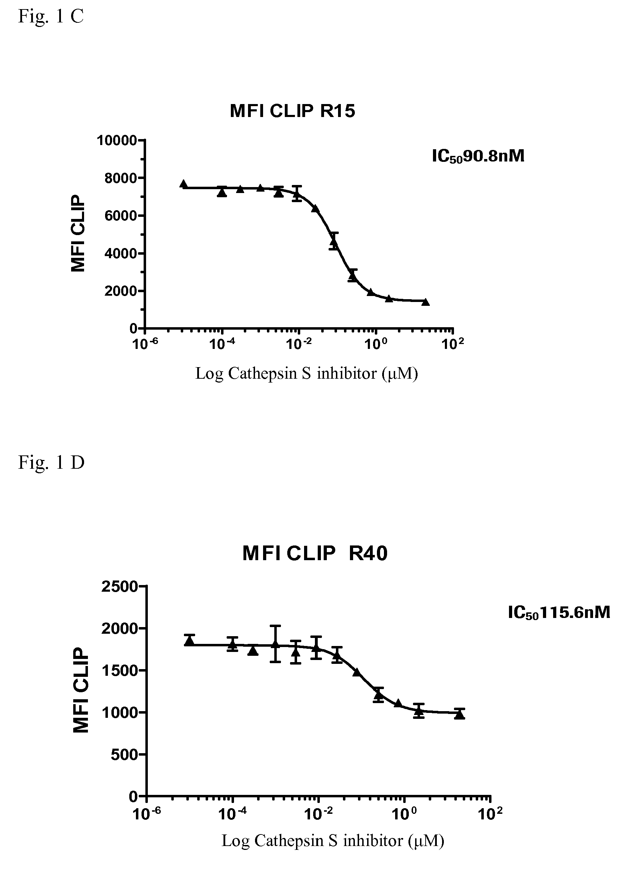

Figure 1A-D: CLIP surface expression on B cells. Whole blood has been incubated for 14 hours with varying concentrations of Cathepsin S inhibitor or carrier. CLIP surface expression on B cells (CD19+) was then determined via flow cytometry. The figure shows the duplicate analysis of four different healthy volunteers (R31, R51, R15 and R40). Four-parameter-dose-response curves and IC50 values have been calculated with GraphPad Prism software. MFI = Mean Fluorescence Intensity.

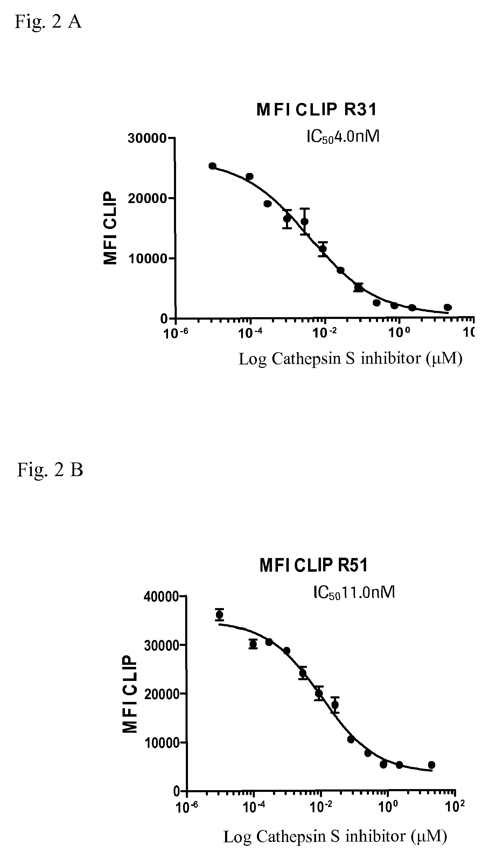

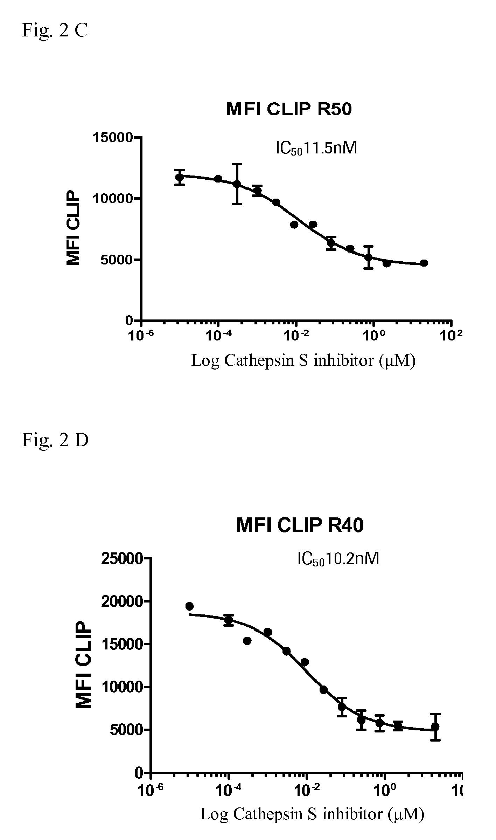

Figure 2A - D: CLIP surface expression on Monocytes. Whole blood has been incubated for 14 hours with varying concentrations of Cathepsin S inhibitor or carrier. The blood has been costimulated with IFNγ and GM-CSF. Subsequently, flow cytometric analysis of CLIP surface expression on Monocytes (CD14+) was performed. The figure shows the duplicate analysis of four different healthy volunteers (R31, R51, R50 and R40). Four-parameter-dose-response curves and IC50 values have been calculated with GraphPad Prism software.

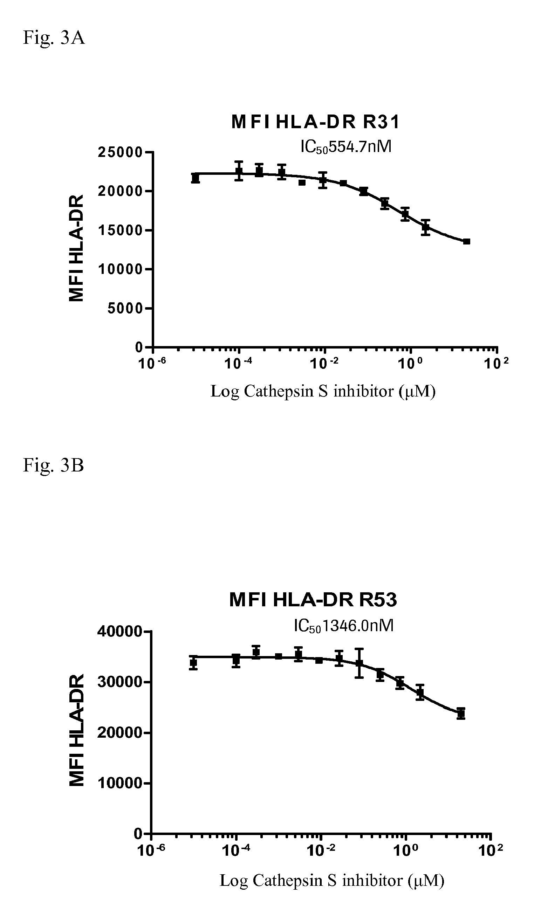

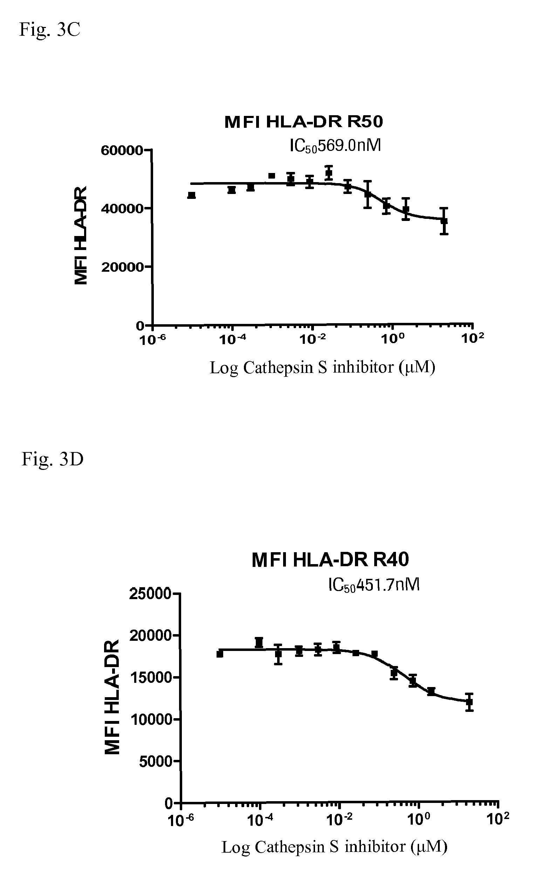

Figure 3A - D: MHC class II cell surface expression on B cells. Whole blood has been incubated for 14 hours with varying concentrations of Cathepsin S inhibitor or carrier. Subsequently, MHC class II surface expression on B cells (CD19+) was determined by flow cytommetry. The figure shows the duplicate analysis of four different healthy volunteers (R31, R51, R15 and R40). Four-parameter-dose-response curves and IC50 values have been calculated with GraphPad Prism software.

### Experimental part

FACS whole blood assay with Cathepsin S inhibitor
- use a sterile Polypropylene 96-well U-bottom plate
- add 100 µl complete RPMI ± Cathepsin S inhibitor
- add 125 µl LI-Heparin whole blood to each tube and mix carefully
- preincubate 30 minutes at 37°C
- add 25 µl complete RPMI ± IFN-γ and GM-CSF to each well
- incubate 13h and 30 minutes at 37°C
unstimulated: DMSO
Cpd (0, 0.0001, 0.0003, 0.001, 0.003, 0.009, 0.027, 0.08, 0.25, 0.74, 2.2 and 20 µM)
stimulated :DMSO
+IFN-γ 400 ng/ml (stock 100 µg/ml, 1:250)
+ GM-CSF 400 ng/ml (stock 100 µg/ml, 1:250)
Cpd (0, 0.0001, 0.0003, 0.001, 0.003, 0.009, 0.027, 0.08, 0.25, 0.74, 2.2 and 20 µM)
+IFN-γ 400 ng/ml (stock 100 µg/ml, 1:250)
+ GM-CSF 400 ng/ml (stock 100 µg/ml, 1:250)
FACS staining:
- prepare 1x lysing solution
- add 1.8 ml 1x lysing solution to each well of a Polypropylene 96-well deep-well plate and prewarm it to 37°C
- add the antibodies (comp. control) or antibody mixture (no predilution in BD stain buffer) to a Polypropylene 96-well V-bottom plate
- add 200 µl whole blood of each sample to the prepared V-bottom plate and mix carefully
- incubate for 30 min on ice in the dark
- transfer 200 µl stained whole blood of each sample to the prepared deep-well plate containing the prewarmed lysis buffer and mix carefully
- incubate at RT for 15 minutes in the dark
- centrifuge 250 x g for 5 minutes and +4°C, remove supernatant
- repeat the cell lysing procedure 1x with 1x lysis buffer prewarmed to room temperature
- wash the cells 1x with 2 ml BD stain buffer (250 x g, 5 minutes, +4°C), remove supernatant
Faser kit-FITC staining in the deep-well plate:
- resuspend the cell pellet in 80 µl BD stain buffer
- add 20 µl FC-blocking reagent and 10 µl FITC-Activator and mix
- incubate for 10 minutes at +4°C
- wash 1x with 2 ml cold BD stain buffer (250 x g, 5 minutes, +4°C) remove supernatant
- resuspend the cell pellet in 80 µl BD stain buffer
- add 20 µl FC-blocking reagent and 10 µl FITC- Enhancer and mix
- incubate for 10 minutes at +4°C
- transfer the cells to FACS tubes via a cell filter and flush the filter with 2 ml PBS
- centrifuge 250 x g for 5 minutes and +4°C, remove supernatant
- resuspend pellet in 200 µl 1x cell fix buffer, vortex carefully
- keep the cells in the dark until the analysis on the FACS

### Antibodies:

| | |
|---|---|
| CLIP FITC (BD, order ID: 555981) | 20 µl/sample |
| | |
| CD14 PerCp-Cy5.5 (BD, order ID: 550787) | 5 µl/sample |
| | |
| CD19 PE (BD, order ID: 555413) | 20 µl/sample |
| | |
| HLA-DR APC (BD, order ID: 347403) | 1.25 µl/sample |

### Materials and buffers:

96-well plates, V-bottom (Nunc, order ID: 249944) Storage: room temperature, non sterile, non tissue culture treated

96-well deep well plates, 2.2 ml (Thermo Scientific, order ID: AB-0661) Storage: room temperature. Non sterile, non tissue culture treated

TopSeal-A 96-well adhesive plate sealing film (PerkinElmer, order ID: 6005185). Storage: room temperature

10x BD Pharm Lyse cell lysis buffer (BD, order ID: 555899). Storage: +4°C. Dilute the 10x BD Pharm Lyse buffer with H₂O bidest. to 1x and prewarm it to the required temperature prior to use

1x BD stain buffer containing 2% FCS (BD, order ID: 554656). Storage: +4C the 1x BD stain buffer containing 2% FCS is ready to use. Keep the BD stain buffer on ice while using

1x PBS without CaCl₂ and without MgCl₂ (Gibco, order ID: 14190-094). Storage: room temperature. The PBS is ready to use. Precool the PBS on ice prior to use

5 ml Polystyrene round-bottom tube (FACS tube, BD, order ID: 352052). Storage: room temperature. Ready to use.

70 µM cup Filcon cell suspension filter (BD, order ID: 340636) Storage: room temperature. Ready to use.

10x BD CellFix cell fixation buffer (BD, order ID: 340181). Storage: room temperature. Dilute the 10x BD CellFix buffer with H₂O bidest. to 1x prior to use.

## Claims

1. A method for monitoring Cathepsin S inhibitor activity in a tissue sample of animals comprising:
a) providing a tissue sample of an animal to whom a Cathepsin S inhibitor has been administered or providing a tissue sample of an animal that was contacted *in vitro* with a Cathepsin S inhibitor, wherein the tissue sample comprise white blood cells,
b) measuring CLIP peptide level and/or MHC II polypeptide level in the white blood cells of the tissue sample of step a) and
c) correlating CLIP peptide level and/or MHC II polypeptide level in the white blood cells to Cathepsin S inhibitor dose, wherein a Cathepsin S inhibitor leads to decreased level of CLIP peptide and/or a decreased level of MHC II polypeptide in the white blood cells.

2. A method for the identification of a Cathepsin S inhibitor comprising:
a) contacting a non-human animal with a test compound,
b) providing a tissue sample of the animal of step a) comprising white blood cells,
c) measuring CLIP peptide level and/or MHC II polypeptide level in the white blood cells of the tissue sample of step b),
wherein a decreased level of CLIP peptide and/or a decreased level of MHC II polypeptide in the white blood cells in the sample of step a) compared to a level of CLIP peptide and/or a level of MHC II polypeptide in white blood cells in a tissue sample of an untreated animal is indicative for a Cathepsin S inhibitor.

3. The method of claim 1 or 2, wherein the tissue sample is blood.

4. The method of claim 1 to 3, wherein the level of CLIP peptide and/or MHC II polypeptide on the cell surface of the white blood cells is measured.

5. The method of claim 4, wherein the level of CLIP peptide and/or MHC II polypeptide on the cell surface of the white blood cells is measured by Flow Cytometry.

6. The method of claim 1 to 5, wherein the white blood cells are Antigen Presenting Cells (APCs), preferably B cells, monocytes, macrophages and dendritic cells.

7. The method of claim 6, wherein the APCs are selected from B cells and monocytes.

8. The method of claim 1 and 3-7, wherein the animal is a human being.

9. The method of claim 2 -7, wherein the non-human animal is a rodent, preferably a rat or mouse.


SEQUENCE LISTING

<110>  F. Hoffmann-La Roche AG

<120>  Method for monitoring Cathepsin S inhibition

<130>  26965 EP

<160>  3

<170>  PatentIn version 3.5

<210>  1
<211>  331
<212>  PRT
<213>  Homo sapiens

<400>  1

<210>  2
<211>  296
<212>  PRT
<213>  Homo sapiens

<400>  2

Met His Arg Arg Arg Ser Arg Ser Cys Arg Glu Asp Gln Lys Pro Val
1               5                   10                  15

Met Asp Asp Gln Arg Asp Leu Ile Ser Asn Asn Glu Gln Leu Pro Met
            20                  25                  30

Leu Gly Arg Arg Pro Gly Ala Pro Glu Ser Lys Cys Ser Arg Gly Ala
        35                  40                  45

Leu Tyr Thr Gly Phe Ser Ile Leu Val Thr Leu Leu Leu Ala Gly Gln
    50                  55                  60

Ala Thr Thr Ala Tyr Phe Leu Tyr Gln Gln Gln Gly Arg Leu Asp Lys
65                  70                  75                  80

Leu Thr Val Thr Ser Gln Asn Leu Gln Leu Glu Asn Leu Arg Met Lys
                85                  90                  95

Leu Pro Lys Pro Pro Lys Pro Val Ser Lys Met Arg Met Ala Thr Pro
            100                 105                 110

Leu Leu Met Gln Ala Leu Pro Met Gly Ala Leu Pro Gln Gly Pro Met
        115                 120                 125

Gln Asn Ala Thr Lys Tyr Gly Asn Met Thr Glu Asp His Val Met His
    130                 135                 140

Leu Leu Gln Asn Ala Asp Pro Leu Lys Val Tyr Pro Pro Leu Lys Gly
145                 150                 155                 160

Ser Phe Pro Glu Asn Leu Arg His Leu Lys Asn Thr Met Glu Thr Ile
                165                 170                 175

Asp Trp Lys Val Phe Glu Ser Trp Met His His Trp Leu Leu Phe Glu
            180                 185                 190

Met Ser Arg His Ser Leu Glu Gln Lys Pro Thr Asp Ala Pro Pro Lys
        195                 200                 205

Val Leu Thr Lys Cys Gln Glu Glu Val Ser His Ile Pro Ala Val His
    210                 215                 220

Pro Gly Ser Phe Arg Pro Lys Cys Asp Glu Asn Gly Asn Tyr Leu Pro
225                 230                 235                 240

Leu Gln Cys Tyr Gly Ser Ile Gly Tyr Cys Trp Cys Val Phe Pro Asn
                245                 250                 255

Gly Thr Glu Val Pro Asn Thr Arg Ser Arg Gly His His Asn Cys Ser
            260                 265                 270

Glu Ser Leu Glu Leu Glu Asp Pro Ser Ser Gly Leu Gly Val Thr Lys
        275                 280                 285

Gln Asp Leu Gly Pro Val Pro Met
    290                 295

<210>  3
<211>  24
<212>  PRT
<213>  Homo sapiens

<400>  3

Leu Thr Val Thr Ser Gln Asn Leu Gln Leu Glu Asn Leu Arg Met Lys
1               5                   10                  15

Leu Pro Lys Pro Pro Lys Pro Val
            20